# EUROPEAN PATENT SPECIFICATION

(11) **EP 2 139 552 B1**
(45) Date of publication and mention of the grant of the patent: **25.05.2011**
(21) Application number: 07716089.3
(22) Date of filing: 26.03.2007
(51) Int. Cl.: A61N 1/05

(54) **A SUTURE SLEEVE FOR IMPLANTING ONE OR MORE ELECTRICAL LEADS INTO A VEIN**
NAHTHÜLLE ZUR IMPLANTATION VON EINER ODER MEHR ELEKTRISCHEN LEITUNGEN IN EINER VENE
MANCHON DE SUTURE D'IMPLANTATION D'UN OU DE PLUSIEURS CONDUCTEURS ÉLECTRIQUES DANS UNE VEINE

(43) Date of publication of application: 06.01.2010
(73) Proprietor: St. Jude Medical AB, 175 84 Järfälla (SE)
(72) Inventor: ECKERDAL, Johan, SE-741 92 Knivsta (SE); HILL, Rolf, S-175 55 Järfälla (SE)
(86) International application number: PCT/SE2007/000294
(87) International publication number: WO 2008/118043

(56) References cited:
- US-A- 5 107 856
- US-A- 5 603 730
- US-A1- 2004 254 623
- US-A1- 2005 137 664

## Description

The invention relates to a suture sleeve for one or more implantable leads, the suture sleeve being adapted to be inserted into a vein to secure and protect the one or more leads from damage when a suture thread is positioned and tied around the vein in the region over the suture sleeve to secure the suture sleeve and prevent bleeding from the vein, comprising two or more lead receiving through holes, into each of which a medical implantable lead may be inserted.

### Background of the invention

It is sometimes desirable to implant an electrical lead into a vein in an animal or human body, such that the lead extends out from the vein. This is the case e.g. when implanting a pacemaker into a body for monitoring and controlling the heart function. The pacemaker itself is implanted just under the skin in a pacemaker pocket at a suitable position, whereas one or more leads are inserted through an opening in the wall of a vein and pushed in until a distal end enters the heart where it can be attached to the heart wall.

However, the vein must be closed after lead insertion to avoid bleeding and the lead must be fixated in relation to the vein to eliminate the risk that the lead accidentally can be drawn out from its position. Avoiding bleeding is very important to prevent local haematoma since haematoma is considered as a major positive predictor for infection in the pacemaker pocket. This is accomplished by means of a suture thread, which is positioned around the vein and the lead in the area of the cut opening in the wall of the vein and which is tied around the vein and the lead. However, when closing the vein by tying the suture around the vein and the lead, there is a risk that the lead might get damaged by squeezing of the suture thread around the lead if the suture is made too tight. The suture stress will be concentrated to a small area and may cause intensive abrasion load to the lead body. On the other hand, if the suture is not tight enough, bleeding may occur and cause haematoma. If the suture is not combined with fixation of the lead elsewhere, there is also a risk for longitudinal lead movement and lead dislodgment if the suture is not sufficient tight.

To eliminate these risks it is known to use a so called suture sleeve, which is positioned around the lead in the area of the lead-through in the wall of the vein. In this way the suture sleeve will protect the lead from damage by the suture thread and the suture sleeve will fixate the lead in a sufficient degree.

However, sometimes it is desirable to connect the pacemaker with the heart by means of two separate leads. In such a case it is common practice to tying the suture around the vein directly onto unprotected leads to prevent from bleeding. The leads are then fixated separately some distance from the vein entrance by using the suture sleeves to prevent from longitudinal lead movement and lead dislodgement. This has to effect that the leads in the venous entrance, where they are unprotected from the suture thread, may become damaged in accordance with the above said.

In US 5107856 is disclosed a suture sleeve for two leads and another for three leads. The suture sleeve is formed as a flexible strip having two or three spaced apart lead receiving channels. During use, one lead is positioned in each of the lead receiving channels and then the flexible strip is wrapped around the leads. Finally, the suture sleeve is positioned in the cut opening in the vein and tied around by a suture thread.

However, there are several disadvantages with such a suture sleeve. For example, it is not possible to position and fixate only one or two leads in a suture sleeve, which is adapted for three leads, since then blood will leak out through the channel which has no lead positioned therein. I.e. when inserting two leads into a vein it is necessary to use a suture sleeve being adapted for exactly two leads, and when inserting three leads into the vein it is necessary to use a suture sleeve being adapted for exactly three leads. Accordingly, it is necessary to keep in stock several types of suture sleeves to be prepared for different applications. Moreover, the leads in question are very small having a cross sectional dimension of only about 2 mm, which has to effect that also the suture sleeve will be very small. This will have to result that it is very difficult to wrap around the leads properly with the suture sleeve, insert it through the opening in the vein and fixate the assembly by means of a suture thread in a correct position without losing the suture sleeve during the handling. With a suture sleeve of this kind it is also impossible to completely eliminate bleeding since, as is evident from the drawings in the document, there will always remain some gaps between the leads and the suture sleeve in an assembled state.

### Summary of the invention

It is an object of the invention to overcome the disadvantages with prior art suture sleeves. More precisely it is an object to provide a suture sleeve for one or more electrical leads which is easy to handle and by means of which it is possible to effectively eliminate any bleeding independently of whether there are leads positioned in every through hole or not. At least this object is achieved by a suture sleeve according to claim 1.

The basis of the invention is the insight that the above object may be achieved by means of a suture sleeve having two or more lead receiving through holes. At least all the holes except one are provided with sealing means, which are easy to break or remove when it is desirable to insert a lead into a specific hole. Accordingly, as long as no lead is inserted into a hole and the sealing means is intact, the sealing means prevents any blood leakage through that hole.

Within this general idea, the invention can be modified in many different ways. In a hereinafter described and in the drawings illustrated embodiment of the invention, the sealing means is in form of a thin membrane, of e.g. silicone, which easily can be broken. However, the sealing means also could be in any other suitable form, e.g. as a removable plug or the like in the hole. Normally, this is not a preferred embodiment, since small removable items in connection with an open cut in a body, could constitute a risk that the item is lost in the cut. The membrane and the plug or the like, could be manufactured as a separate detail and attached in the hole of the suture sleeve by means of for example an adhesive. However, it is preferred to make the sealing means in the same production step as the suture sleeve itself, e.g. by injection moulding.

The through holes in the suture sleeve can be made as complete and unbroken holes, in which case the leads are inserted by displacing the leads in the axial direction of each hole. In such a case a sealing means in form of a membrane can be broken by means of the lead when it is inserted into the hole. A prerequisite for forming the suture sleeve in this way is that the lead is substantial isodiametric along its entire length.

However, in many cases the leads have a thickened portion, e.g. in their tip portions. For this reason, according to a hereinafter described embodiment, at least all of the holes except one are formed with a slot along the entire length of the hole and the suture sleeve is formed of an elastic material. In this way a lead can be inserted into a through hole by deflecting the material around the hole, for widening the slot, and inserting the lead sideways through the widened slot. It is, in such a case, favourable if the slot formed through holes, are each provided with a sealing means in form of a membrane, which will brake when deflecting the material around the hole and widening the slot. However, one of the through holes can, as in the described and illustrated embodiment, be unbroken and adapted to be pre-assembled onto a lead. In this way there is no risk of losing the suture sleeve during an implantation.

Moreover, the suture sleeve could be manufactured with an arbitrary number of holes, however at least two. Normally it is sufficient to manufacture the suture sleeve with three holes since this will cover most application ranges for pacemakers. Then it is possible to attach one, two or three electrical leads, as desired, in a secured and bleeding preventing way.

### Brief description of the drawings

Hereinafter an embodiment of the invention will be described by way of example with reference to the drawing, in which:
- Fig 1: is a perspective view of suture sleeve accord- ing to the invention;
- Fig 2: is an end view of the suture sleeve in fig 1;
- Fig 3: is an end view according to fig 2 with the ma- terial around a slot in one of the through holes deflected outwards; and
- Fig 4: is a perspective view showing the suture sleeve, with two electrical leads in it, in- serted into an opening in a vein and tied around.with a suture thread.

### Detailed description of an embodiment of the invention

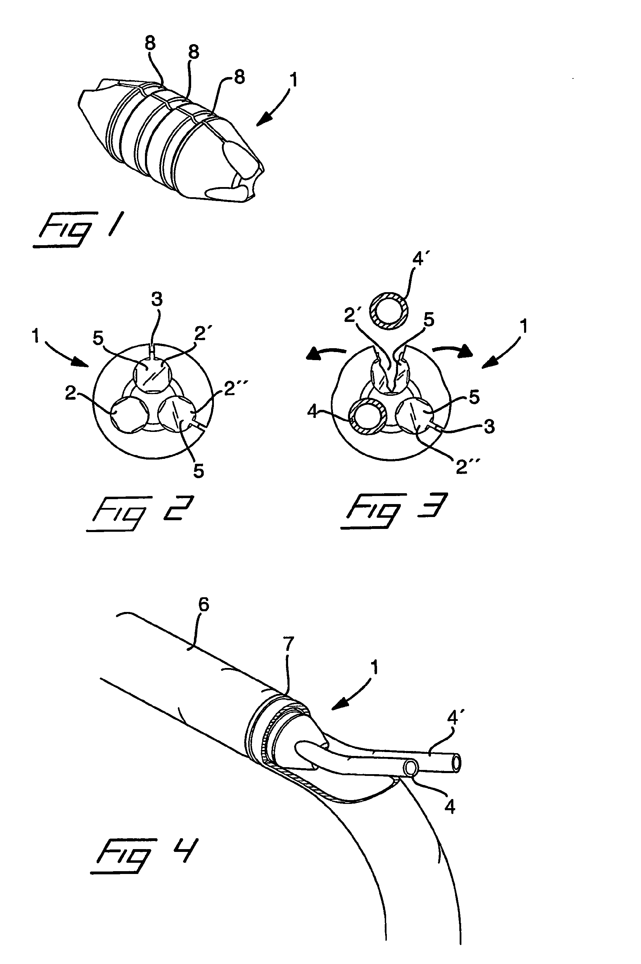

Reference is first made to figs 1 and 2 of the drawings, in which a suture sleeve 1 according to the invention is shown in a perspective view and an end view, respectively. The suture sleeve has an elongated form with a generally circular cross section and is provided with a truncated conical shape in each end to facilitate introducing into an opening in a vein.

The suture sleeve comprises three through holes 2, 2' and 2" in its longitudinal direction. As evident from fig 2, one of the through holes 2 is unbroken, whereas the other through holes 2' and 2" are formed with a slot 3 in its longitudinal direction. The reason for forming the through holes in this way is that, as is illustrated in fig 3, the suture sleeve is adapted to be pre-assembled onto one electrical lead 4 such that the lead extends through the unbroken through hole 2. In this way the suture sleeve will be held safely on the lead without any risk for it to be lost during implantation. Subsequently one or two additional leads 4' can optionally be mounted in the suture sleeve by, as is illustrated in fig 3, simply deflecting the material around the slot 3 such that the slot is widened and a lead can be inserted into the through hole 2' sideways.

Each of the through holes 2', 2" being formed with a slot 3, is provided with a sealing means in form of a thin membrane 5, which blocks the through hole. When mounting a lead into one of these through holes, the membrane will break as the slot is widened by deflecting the material around the slot, as is illustrated in fig 3.

In fig 4 is illustrated a situation where a suture sleeve 1 is partly inserted into an opening in a vein 6. Two leads 4, 4' are inserted into the vein via a respective through hole 2, 2' in the suture sleeve. Although the third through hole 2" does not accommodate any lead, bleeding is prevented from this through hole owing to the fact that the membrane 5 still is intact in this hole. A suture thread 7 is positioned and tied around the vein 6 and the suture sleeve 1. In this way bleeding is prevented in the region between the suture sleeve and the wall of the vein. In order to improve the sealing and fixation between the suture sleeve and the vein, the suture sleeve is formed with circumferential grooves 8 around its outer periphery, as is best seen in fig 1, in which the suture thread 7 and vein tissue may sink into when tying the suture thread.

## Claims

1. A suture sleeve for one or more implantable leads (4, 4'), the suture sleeve (1) being adapted to be inserted into a vein (6) to secure and protect the one or more leads from damage when a suture thread (7) is positioned and tied around the vein in the region over the suture sleeve to secure the suture sleeve and prevent bleeding from the vein, comprising two or more lead receiving through holes (2, 2', 2"), into each of which a medical implantable lead may be inserted, **characterized in that** the suture sleeve (1) is formed such that all of the holes except an unsealed one are provided with sealing means (5), which easily can be broken or removed when inserting a lead into the hole such that, when inserted into a vein (6), bleeding is prevented from a through hole of the sleeve by means of the sealing means even if no lead is positioned in the hole.

2. A suture sleeve according to claim 1, **characterized in that** the sealing means comprises a thin membrane (5), which is positioned in the hole (2', 2") and is easily breakable.

3. A suture sleeve according to claim 1 or 2, **characterized in that** the suture sleeve (1) is formed of an elastic material and such that at least all of the through holes (2', 2") except one is formed with a slot (3) in the longitudinal direction of the hole, such that the material around the hole can be deflected for widening the slot and inserting a lead sideways into the hole through the widened slot, and at least each of the holes being cut through by slots is provided with sealing means (5), which is breakable or removable when opening and widening the slot.

4. A suture sleeve according to any of the preceding claims, **characterized in that** all of the through holes (2, 2', 2") are provided with sealing means (5).

5. A suture sleeve according to claim 3 or 4, **characterized in that** all of the through holes (2, 2', 2") are provided with a slot (3).

6. A suture sleeve according to claim 3, **characterized in that** the suture sleeve (1) is formed such that one of the through holes (2) is unbroken and adapted to be pre-assembled onto one of the leads.

7. A suture sleeve according to any of the preceding claims, **characterized in that** the suture sleeve (1) is formed as an elongated sleeve body having two or more lead receiving through holes (2, 2', 2").

8. A suture sleeve according to any of the preceding claims, **characterized in that** the suture sleeve (1) has a generally circular cross section.

9. A suture sleeve according to any of the preceding claims, **characterized in that** it comprises one or more circumferential grooves (8) around its outer periphery.

10. A suture sleeve according to any of the preceding claims, **characterized in that** the suture sleeve (1) has a truncated conical form.

## Patentansprüche

1. Nahthülle für eine oder mehr implantierbare Leitung/en (4, 4'), wobei die Nahthülle (1) angepasst ist, in eine Vene (6) eingesetzt zu werden, um die eine oder mehr Leitung/en vor Beschädigung zu sichern und zu schützen, wenn ein Nahtfaden (7) um die Vene in dem Bereich über der Nahthülle positioniert und festgezogen wird, um die Nahthülle zu sichern und ein Bluten aus der Vene zu verhindern, umfassend zwei oder mehr leitungsaufnehmende Durchgangslöcher (2, 2', 2"), in die jeweils eine medizinische implantierbare Leitung eingesetzt werden kann, **dadurch gekennzeichnet, dass** die Nahthülle (1) so gebildet ist, dass alle Löcher, ausgenommen ein unabgedichtetes, mit einem Abdichtmittel (5) versehen sind, das einfach zerstört oder entfernt werden kann, wenn eine Leitung in das Loch eingesetzt wird, so dass, wenn es in eine Vene (6) eingesetzt ist, das Bluten von einem Durchgangsloch der Hülle mittels des Abdichtmittels verhindert wird, selbst wenn keine Leitung in dem Loch positioniert ist.

2. Nahthülle nach Anspruch 1, **dadurch gekennzeichnet, dass** das Abdichtmittel eine dünne Membran (5) umfasst, die in dem Loch (2', 2") positioniert ist und einfach zerstörbar ist.

3. Nahthülle nach Anspruch 1 oder 2, **dadurch gekennzeichnet, dass** die Nahthülle (1) aus einem elastischen Werkstoff gebildet ist und so, dass zumindest alle der Durchgangslöcher (2', 2"), ausgenommen eines, mit einem Schlitz (3) in Längsrichtung des Lochs gebildet sind, so dass der Werkstoff um das Loch umgebogen werden kann, um den Schlitz auszuweiten und eine Leitung seitwärts in das Loch in den ausgeweiteten Schlitz einzusetzen, und mindestens jedes der Löcher, die von Schlitzen durchtrennt sind, mit einem Abdichtmittel (5) versehen ist, das beim Öffnen und Ausweiten des Schlitzes zerstörbar oder entfernbar ist.

4. Nahthülle nach einem der vorhergehenden Ansprüche, **dadurch gekennzeichnet, dass** alle der Durchgangslöcher (2, 2', 2") mit einem Abdichtmittel (5) versehen sind.

5. Nahthülle nach Anspruch 3 oder 4, **dadurch gekennzeichnet, dass** alle der Durchgangslöcher (2, 2', 2") mit einem Schlitz (3) versehen sind.

6. Nahthülle nach Anspruch 3, **dadurch gekennzeichnet, dass** die Nahthülle (1) so gebildet ist, dass eines der Durchgangslöcher (2) unzerstört ist und angepasst ist, auf einer der Leitungen vormontiert zu werden.

7. Nahthülle nach einem der vorhergehenden Ansprüche, **dadurch gekennzeichnet, dass** die Nahthülle (1) als länglicher Nahtkörper gebildet ist, der zwei oder mehr leitungsaufnehmende Durchgangslöcher (2, 2', 2") aufweist.

8. Nahthülle nach einem der vorhergehenden Ansprüche, **dadurch gekennzeichnet, dass** die Nahthülle (1) einen allgemein kreisförmigen Querschnitt aufweist.

9. Nahthülle nach einem der vorhergehenden Ansprüche, **dadurch gekennzeichnet, dass** sie eine oder mehr Umfangsnuten (8) um ihren äußeren Umfang aufweist.

10. Nahthülle nach einem der vorhergehenden Ansprüche, **dadurch gekennzeichnet, dass** die Nahthülle (1) eine kegelstumpfartige Form aufweist.

## Revendications

1. Manchon de suture pour un ou plusieurs conducteurs implantables (4, 4'), le manchon de suture (1) étant adapté pour être inséré dans une veine (6) pour fixer et protéger le ou les plusieurs conducteurs de dommages quand un fil de suture (7) est positionné et attaché autour de la veine dans la région au-dessus du manchon de suture pour fixer le manchon de suture et empêcher un saignement de la veine, comprenant deux ou plus de trous de passage de réception de conducteur (2, 2', 2"), dans chacun desquels un conducteur implantable médical peut être inséré, **caractérisé en ce que** le manchon de suture (1) est formé de telle sorte que tous les trous, sauf un non étanche, sont pourvus d'un moyen étanchéifiant (5), qui peut être aisément rompu ou retiré lors de l'insertion d'un conducteur dans le trou de sorte que, quand il est inséré dans une veine (6), un saignement est empêché depuis un trou de passage du manchon au moyen du moyen étanchéifiant même si aucun conducteur n'est positionné dans le trou.

2. Manchon de suture selon la revendication 1, **caractérisé en ce que** le moyen étanchéifiant comprend une fine membrane (5), qui est positionnée dans le trou (2', 2") et peut aisément être rompue.

3. Manchon de suture selon la revendication 1 ou 2, **caractérisé en ce que** le manchon de suture (1) est formé d'un matériau élastique et de telle sorte qu'au moins tous les trous (2', 2") sauf un sont formés avec une fente (3) dans la direction longitudinale du trou, de sorte que le matériau autour du trou peut être dévié pour élargir la fente et insérer un conducteur latéralement dans le trou à travers la fente élargie, et au moins chacun des trous étant coupés par des fentes est pourvu d'un moyen étanchéifiant (5), qui peut être rompu ou retiré lors de l'ouverture et de l'élargissement de la fente.

4. Manchon de suture selon l'une quelconque des revendications précédentes, **caractérisé en ce que** tous les trous de passage (2, 2', 2") sont pourvus d'un moyen étanchéifiant (5).

5. Manchon de suture selon la revendication 3 ou 4, **caractérisé en ce que** tous les trous de passage (2, 2', 2") sont pourvus d'une fente (3).

6. Manchon de suture selon la revendication 3, **caractérisé en ce que** le manchon de suture (1) est formé de telle sorte que l'un des trous de passage (2) n'est pas rompu et est adapté pour être préassemblé sur l'un des conducteurs.

7. Manchon de suture selon l'une quelconque des revendications précédentes, **caractérisé en ce que** le manchon de suture (1) est formé en tant que corps de manchon allongé présentant deux ou plusieurs trous de passage de réception de conducteur (2, 2', 2").

8. Manchon de suture selon l'une quelconque des revendications précédentes, **caractérisé en ce que** le manchon de suture (1) présente une coupe transversale généralement circulaire.

9. Manchon de suture selon l'une quelconque des revendications précédentes, **caractérisé en ce qu'**il comprend une ou plusieurs rainures circonférentielles (8) autour de sa périphérie extérieure.

10. Manchon de suture selon l'une quelconque des revendications précédentes, **caractérisé en ce que** le manchon de suture (1) présente une forme conique tronquée.
